**Europäisches Patentamt**

**European Patent Office**

**Office européen des brevets**

(19)

(11) Veröffentlichungsnummer: **0 249 825 B1**

# (12) EUROPÄISCHE PATENTSCHRIFT

(45) Veröffentlichungstag der Patentschrift: **06.11.91**

(51) Int. Cl.⁵: **A61B 5/05**, A61N 1/365

(21) Anmeldenummer: **87108122.0**

(22) Anmeldetag: **04.06.87**

Die Akte enthält technische Angaben, die nach dem Eingang der Anmeldung eingereicht wurden und die nicht in dieser Patentschrift enthalten sind.

(54) **Vorrichtung zur Impedanzmessung an Körpergeweben.**

(30) Priorität: **16.06.86 DE 3620276**

(43) Veröffentlichungstag der Anmeldung:
**23.12.87 Patentblatt 87/52**

(45) Bekanntmachung des Hinweises auf die
Patenterteilung:
**06.11.91 Patentblatt 91/45**

(84) Benannte Vertragsstaaten:
**DE FR GB IT NL SE**

(56) Entgegenhaltungen:
**EP-A- 0 074 126**
**GB-A- 2 070 282**
**US-A- 3 532 086**
**US-A- 3 608 542**
**US-A- 3 994 284**

(73) Patentinhaber: **SIEMENS AKTIENGESELL-
SCHAFT**
**Wittelsbacherplatz 2**
**W-8000 München 2(DE)**

(72) Erfinder: **Heinze, Roland**
**Simbacher Strasse 9**
**W-8000 München 80(DE)**
Erfinder: **Stangl, Karl, Dr.**
**Landwehrstrasse 7**
**W-8000 München 2(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung bezieht sich auf eine Vorrichtung zur Impedanzmessung an Körpergeweben gemäß Oberbegriff des Patentanspruchs 1.

Eine solche Vorrichtung mit einer Auswerteeinrichtung im Sinne der Heraustrennung lediglich von höherfrequenten Signalanteilen ist z.B. im Zusammenhang mit frequenzgesteuerten Herzschrittmachern durch die US-PS 43 03 075 vorbekannt Eine weitere solche Vorrichtung mit einer Auswerteeinrichtung im Sinne der Heraustrennung sowohl von niederfrequenten als auch höherfrequenten Signalanteilen ist im Zusammenhang mit der Bestimmung Blutverlusten bei einer Operation z.B. dur die US-PS 35 32 088 vorbekannt. Der niederfrequente Anteil ist ein Maß für das Blutvolumen.

Impedanzmessungen im Körpergewebe (einschließlich Blut) dienten bisher zur Bestimmung von mechanischen Volumenänderungen des Körpers, z.B. des Schlagvolumens des Herzens sowie auch der Atmung aufgrund Thoraxbewegungen. Die Änderung der Impedanz kann zur Frequenzsteuerung von Herzschrittmachern verwendet werden. Grundlage der Impedanzmessung ist vereinfacht die folgende physikalische Beziehung:

$$R = \frac{1}{\sigma_R} \cdot K1 \qquad\qquad (1)$$

wobei R die Impedanz,
$\sigma_R$ der Leitwert

$$\left(\frac{1}{\Omega \cdot cm}\right),$$

$K_1 = \frac{l}{F}$ die der Leitungsstrecke proportionale Größe $(cm^{-1})$ mit
l als wirksamem Elektrodenabstand (cm) und
F als wirksamem Leitungsquerschnitt $(cm^2)$
zwischen den Elektroden ist.

Die Änderungen der Impedanz $\Delta R$ sind ein Maß für die Atmung oder auch ein Maß für das Schlagvolumen des Herzens. Die Messung periodischer Impedanzschwankungen $\Delta R$ erfaßt im wesentlichen die Änderungen $\Delta l$ von l oder $\Delta F$ von F auf der Leitungsstrecke und damit die Änderungen $\Delta K1$. Sie ist aber auch abhängig von Änderungen $\Delta\sigma_R$ des Leitwertes, da nach der Beziehung (1) gilt:

$$R + \Delta R = \frac{1}{\sigma_R + \Delta\sigma_R} \cdot (K1 + \Delta K1) \qquad\qquad (2)$$

Daraus ergibt sich bei Herausfilterung des niederfrequenten Anteils aus dem Impedanzsignal für den durch gelassenen höherfrequenten Anteil die Beziehung:

$$\Delta R \sim \frac{\Delta K1}{\sigma_R + \Delta\sigma_R}, \qquad\qquad (3)$$

so daß sich bei Leitwertänderungen eine gegensinnige Änderung der Impedanz ergibt. Dies führt zu unerwünschten Meßwertverfälschungen.

Aufgabe vorliegender Erfindung ist es, eine Vorrichtung zur Impedanzmessung der eingangs genannten Art dahingehend weiterzubilden, daß sich bei Messung periodischer Impedanzschwankungen keine Meßwertfälschungen aufgrund Beeinflussung durch Leitwertänderung mehr ergeben.

Die Aufgabe wird erfindungsgemäß durch die kennzeichnenden Merkmale des Patentanspruchs 1 gelöst.

Die Erfindung geht aus von der Erkenntnis, daß niederfrequente Signalanteile des Impedanzsignals auch ein Maß für den Leitwert sind (also Leitwertänderungen wiederspiegeln), während bekanntlich die höherfrequenten Signalanteile im wesentlichen Änderungen von I und F wiedergeben. Durch getrennte Erfassung beider Signalanteile können die höherfrequenten Anteile mit dem niederfrequenten Anteil korrigiert und damit also ein Impedanzmeßwert erhalten werden, der von Leitwertänderungen unbeeinflußt und damit sehr viel exakter als ein nach herkömmlichen Verfahren ermittelter Impedanzmeßwert ist.

In vorteilhafter Ausgestaltung der Erfindung wird der korrigierte Meßwert als Steuersignal dem Frequenzsteuerteil eines frequenzgesteuerten Herzschrittmachers zugeführt zur Steuerung der Stimulationsfrequenz in dem Sinne, daß bei sich änderndem Meßwert die Stimulationsfrequenz entsprechend geändert wird.

Weitere Vorteile und Einzelheiten der Erfindung ergeben sich aus der nachfolgenden Beschreibung eines Ausführungsbeispiels anhand der Zeichnung und in Verbindung mit den Unteransprüchen.

Es zeigen:

Figur 1 ein erstes Ausführungsbeispiel der Erfindung im Prinzipschaltbild,

Figur 2 ein zweites Ausführungsbeispiel der Erfindung im Prinzipschaltbild und

Figur 3 die Verwendung der Erfindung gemäß dem Prinzipschaltbild der Figur 1 oder 2 in einem frequenzgesteuerten Herzschrittmacher.

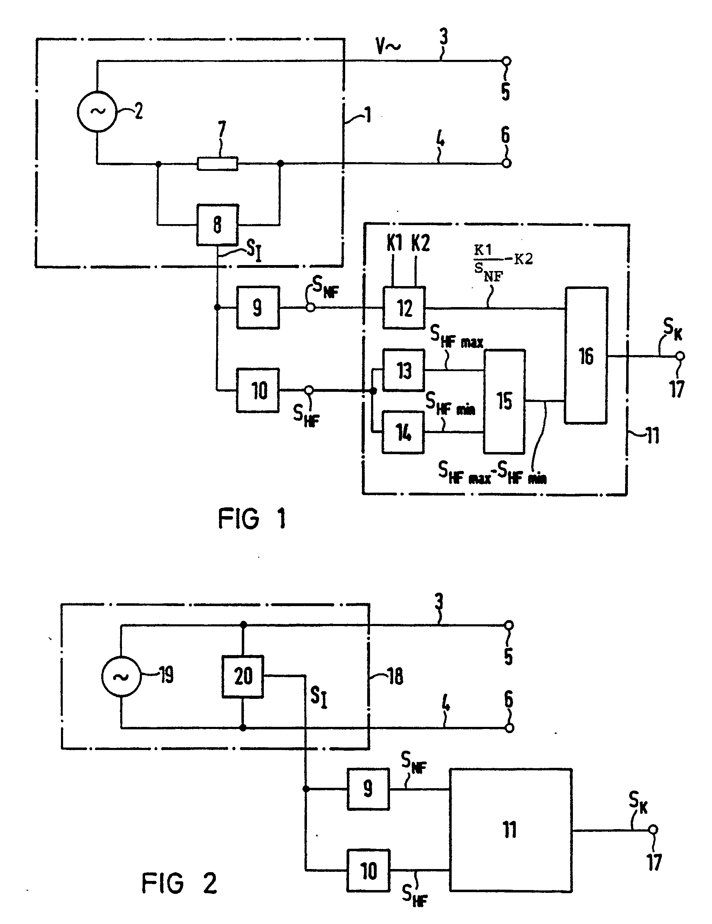

Der Impedanzmesser 1 der Figur 1 umfaßt als Signalquelle zum Einprägen eines elektrischen Signals einen Wechselspannungserzeuger 2 (z.B. 1 kHz-Wechselspannungserzeuger), der über Elektrodenleitungen 3 und 4 mit zugehörigen Elektroden 5 und 6 einem (nicht dargestellten) Körpergewebe eine konstante Wechselspannung V$\sim$ (z.B. 1 kHz-Wechselspannung) einprägt. Die Meßanordnung der Figur 1 ist dabei vorzugsweise zur intrakorporalen Messung ausgelegt. Es sind also zumindest die Elektroden 5 und 6 im Körpergewebe implantiert; vorzugsweise ist jedoch die gesamte Meßeinrichtung implantiert. In Abhängigkeit von der eingeprägten Wechselspannung V$\sim$ wird über einen niederohmigen Serienwiderstand 7 (z.B. 100 Ohm) der vom Strom in den Elektrodenleitungen 3 und 4 verursachte Spannungsabfall mittels eines Spannungsmessers 8 erfaßt.

Das Ausgangssignal des Spannungsmessers 8 wird dann einer Auswerteeinrichtung mit einem Tiefpaßfilter 9 und einem Bandpaßfilter 10 zugeführt. Die obere Grenzfrequenz des Tiefpaßfilters 9 liegt im Bereich 0,1 bis 0,4 Hz. Das Tiefpaßfilter 9 trennt aus dem Ausgangssignal $S_I$ (Impedanzsignal) des Spannungsmessers 8 lediglich die niederfrequenten Signalanteile $S_{NF}$, die dem Leitwert $\sigma_R$ im Körpergewebe entsprechen, heraus.

Das Bandpaßfilter 10, das zur Erfassung der Atmung auf einen Frequenzbereich von 0,2 bis 0,6 Hz oder zur Erfassung des Schlagvolumens des Herzens auf eine Frequenz von 1 bis 3 Hz einstellbar ist, erfaßt aus dem Impedanzsignal $S_I$ die im Bandbereich liegenden höherfrequenten Signalanteile $S_{HF}$ des Impedanzsignals.

Die beiden Signalanteile $S_{NF}$ und $S_{HF}$ werden dann einer Korrektureinrichtung 11 zugeführt, die die höherfrequenten Signalanteile $S_{HF}$ mit den niederfrequenten Signalanteilen $S_{NF}$ im Sinne der Befreiung der höherfrequenten Signalanteile von einer Beeinflussung durch Leitwertänderungen $\Delta \sigma_R$ korrigiert. Zu diesem Zwecke umfaßt die Korrektureinrichtung 11 ein Formglied 12, das die niederfrequenten Signalanteile $S_{NF}$ in Abhängigkeit von eingebbaren Größen K1 und K2 in ein Signal mit der folgenden Beziehung

$$\frac{K1}{S_{NF}} - K2$$

umformt. Die Größe K1 berücksichtigt dabei - wie zuvor schon in der Beziehung (1) beschrieben - die Geometrie der Elektrodenanordnung 5, 6 bei unterschiedlich großen Patienten, während die Größe K2 die Art des Übertragungsweges zwischen den Elektroden 5 und 6 (Art der Körpersubstanz, wie z.B. knochiges oder weicheres Gewebe) berücksichtigt. Die Korrektureinrichtung 11 beinhaltet darüber hinaus eine Spitzendetektoreinrichtung 13, 14 für die Spitzen $S_{HF\ max}$ und $S_{HF\ min}$ der hochfrequenten Signalanteile $S_{HF}$. Ein Differenzbildner 15 bildet die Differenz $S_{HF\ max} - S_{HF\ min}$. In einem Multiplizierer 16 wird vom Signal $S_{HF\ max} - S_{HF\ min}$ mit dem Korrektursignal

$$\frac{K1}{S_{NF}} - K2$$

multipliziert. Das Ergebnis ist ein korrigiertes Signal $S_K$ am Signalausgang 17 der Korrektureinrichtung 11.

In der Figur 2 umfaßt der Impedanzmesser 18 eine Wechselstromquelle 19 (z.B. 1 kHz-Wechselstromquelle), die dem Körpergewebe über die Elektrodenleitungen 3 und 4 sowie den zugehörigen Elektroden 5 und 6 einen konstanten Wechselstrom I~(z.B. 1 kHz-Wechselstrom) einprägt. Das gesamte Meßsystem ist wieder vorzugsweise als intrakorporales Meßsystem ausgelegt. Gemessen wird diesmal die Wechselspannung zwischen den Elektroden 5 und 6 mittels eines parallelgeschalteten Spannungsmessers 20, der einen Dividierer zur Bildung von 1/V~ beinhaltet. Das Ausgangssignal $S_I$ (Impedanzsignal) des Spannnungsmessers 20 wird dann in derselben Weise, wie zuvor im Zusammenhang mit der Figur 1 beschrieben, in einer Filtereinrichtung 9, 10 mit nachgeschalteter Korrektureinrichtung 11 ausgewertet.

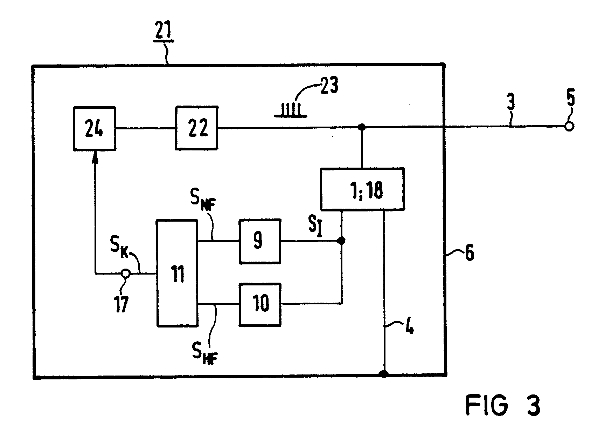

Die Figur 3 zeigt die Anwendung einer Meßanordnung gemäß der Figur 1 oder Figur 2 in einem frequenzgesteuerten Herzschrittmacher 21. Gleiche Bauelemente sind dabei mit gleichen Bezugsziffern versehen. Die Elektrode 5 ist im vorliegenden Fall gleichzeitig die Stimulationselektrode des Herzschrittmachers 21, während die Elektrode 6 durch das leitende (z.B. metallische) Gehäuse des Herzschrittmachers gebildet ist. Die Elektrodenleitung 3 entspricht dem Stimulationskatheter des Herzschrittmachers.

Der frequenzgesteuerte Herzschrittmacher 21 umfaßt einen Pulsgenerator 22 für die Stimulationsimpulse 23. Die Folgefrequenz der Stimulationsimpulse 23 (Stimulationsfrequenz) ist am Pulsgenerator 22 über ein Frequenzsteuerteil 24 in Abhängigkeit vom korrigierten Signal $S_K$ am Ausgang 17 der Korrektureinrichtung 11 steuerbar in dem Sinne, daß bei sich änderndem Signal $S_K$ die Stimulationsfrequenz entsprechend geändert wird. Die Stimulationsfrequenz steigt also, wenn $S_K$ größer wird. Sie sinkt, wenn $S_K$ kleiner wird.

## Patentansprüche

1. Vorrichtung zur Impedanzmessung an Körpergeweben mit einer Signalquelle (2,19) zum Einprägen eines elektrischen Signals in das Körpergewebe, mit einer an dem Körpergewebe angeordneten und Elektroden (5,6) aufweisenden Einrichtung (1,18) zum Erfassen eines Impedanzsignals $S_I$ aus dem Körpergewebe in Abhängigkeit von dem eingeprägten elektrischen Signal und mit einer Auswerteeinrichtung (9,10), die zur Auswertung des Impedanzsignals $S_I$ nieder- und höherfrequenten Signalanteile $S_{NF}$ und $S_{HF}$ aus dem Impedanzsignal $S_I$ heraustrennt, wobei die Auswerteeinrichtung (9,10), ausgehend davon, daß das Impedanzsignal $S_I$ von den Änderungen $\Delta \sigma_R$ des Leitwertes $\sigma_R$ des Körpergewebes und von den Änderungen $\Delta K1$ der Geometrie $K1$ der Elektrodenanordnung (5,6) abhängig ist, zur Heraustrennung der nur von den Änderungen $\Delta \sigma R$ des Leitwertes $\sigma_R$ abhängigen niederfrequenten Signalanteile $S_{NF}$ ausgebildet ist; **dadurch gekennzeichnet** daß eine Korrektureinrichtung (11) vorgesehen ist, die die höherfrequenten Signalanteile $S_{HF}$, die proportional sind zu

$$\frac{\Delta K_1}{\sigma_R + \Delta \sigma_R} ,$$

mittels den niederfrequenten Signalanteilen $S_{NF}$ im Sinne einer Befreiung der höherfrequenten Signalanteile $S_{HF}$ von ihrer Beeinflussung durch die Leitwertänderungen $\Delta \sigma_R$ korrigiert und einen Signalausgang (17) für das korrigierte Signal $S_K$ umfaßt.

2. Vorrichtung nach Anspruch 1, **dadurch gekennzeichnet**, daß die Auswerteeinrichtung (9, 10) ein Tiefpaßfilter (9) für die von den Leitwertänderungen $\Delta \sigma_R$ abhängigen niederfrequenten Signalanteile $S_{NF}$ umfaßt.

3. Vorrichtung nach Anspruch 2, **dadurch gekennzeichnet**, daß das Tiefpaßfilter (9) eine obere Grenzfrequenz im Bereich 0,1 bis 0,4 Hz aufweist.

4. Vorrichtung nach einem der Ansprüche 1 bis 3, **dadurch gekennzeichnet**, daß die Auswerteeinrichtung (9, 10) ein Bandpaßfilter (10) für die höherfrequenten Signalanteile $S_{HF}$ umfaßt.

5. Vorrichtung nach Anspruch 4, **dadurch gekennzeichnet**, daß das Bandpaßfilter (10) zur Erfassung der Atmung auf einen Frequenzbereich von 0,2 bis 0,6 Hz einstellbar ist und daß die obere Grenzfrequenz des Tiefpaßfilters (9) kleiner oder gleich 0,2 Hz ist.

6. Vorrichtung nach Anspruch 4 oder 5, **dadurch gekennzeichnet**, daß das Bandpaßfilter (10) zur Messung des Schlagvolumens des Herzens auf eine Frequenz von 1 bis 3 Hz einstellbar ist.

7. Vorrichtung nach einem der Ansprüche 1 bis 6, **dadurch gekennzeichnet,** daß die Korrektureinrichtung (11) zur Korrektur der höherfrequenten Signalteile $S_{HF}$ mit den niederfrequenten Signalanteilen $S_{NF}$ gemäß der Beziehung

$$\frac{K1}{S_{NF}} - K2$$

ausgebildet ist, wobei K1 eine die Geometrie der Elektrodenanordnung (5,6) berücksichtigende erste Größe und K2 eine die Art des Übertragungsweges zwischen den Elektroden (5,6) berücksichtigende zweite Größe ist.

8. Vorrichtung nach Anspruch 7, **dadurch gekennzeichnet,** daß in der Korrekturvorrichtung (11) ein Formglied (12), das die niederfrequenten Signalanteile $S_{NF}$ nach der Beziehung

$$\frac{K1}{S_{NF}} - K2$$

umformt, ausgangsseitig mit einer Einrichtung (13 - 15) zur Bildung der Maximal/Minimalwert-Differenz $S_{HF\,max} - S_{HF\,min}$ der höherfrequenten Signalanteile $S_{HF}$ im Sinne der Korrekturbildung zusammengeschaltet ist.

9. Vorrichtung nach Anspruch 8, **dadurch gekennzeichnet,** daß das Formglied (12) und die Einrichtung (13 - 15) zur Bildung der Maximal/Minimalwert-Differenz über einen Multiplizierer (16) im Sinne der Bildung des Produktes zwischen der Maximal/Minimalwert-Differenz $S_{HF\,max} - S_{HF\,min}$ und dem Signal

$$\frac{K1}{S_{NF}} - K2$$

verbunden sind.

10. Vorrichtung nach einem der Ansprüche 1 bis 9, **dadurch gekennzeichnet,** daß vom Signalausgang (17) der Auswerteeinrichtung (11) das Korrektursignal $S_K$ als Steuersignal dem Frequenzsteuerteil (24) eines frequenzgesteuerten Herzschrittmachers (21) zugeführt wird zur Steuerung der Stimulationsfrequenz in dem Sinne, daß bei sich änderndem Korrektursignal $S_K$ die Stimulationsfrequenz entsprechend geändert wird.

**Claims**

1. Apparatus for impedance measurement of body tissues, having a signal source (2, 19) for impressing an electrical signal into the body tissue, having a device (1, 18) for detecting an impedance signal $S_I$ from the body tissue as a function of the impressed electrical signal, such a device being arranged on the body tissue and having electrodes (5, 6), and said apparatus having an evaluation device (9, 10) which, for the purpose of evaluating the impedance signal $S_I$, filters low-frequency and higher-frequency signal components $S_{NF}$ and $S_{HF}$ out of the impedance signal $S_I$, in which case, assuming that the impedance signal $S_I$ is dependent on the changes $\Delta\,\sigma_R$ of the conductance value $\sigma_R$ of the body tissue and on the changes $\Delta\,K1$ of the geometry K1 of the electrode arrangement (5, 6), the evaluation device (9, 10) is designed for filtering out the low-frequency signal components $S_{NF}$ which are dependent only on the changes $\Delta\,\sigma_R$ of the conductance value $\sigma_R$; characterised in that a correction device (11) is provided which corrects the higher-frequency signal components $S_{HF}$ which are proportional to

EP 0 249 825 B1

$$\frac{\Delta\ K_x\,1}{\sigma_R\ +\ \Delta\sigma_R}$$

by means of the low-frequency signal components $S_{NF}$ to the effect of freeing the higher-frequency signal components $S_{HF}$ from their influence by the conductance value changes $\Delta\ \sigma_R$ and comprises a signal output (17) for the corrected signal $S_K$.

2. Apparatus according to Claim 1, characterised in that the evaluation device (9, 10) comprises a low-pass filter (9) for the low-frequency signal components $S_{NF}$ which are dependent on the conductance value changes $\Delta\ \sigma_R$.

3. Apparatus according to Claim 2, characterised in that the low-pass filter (9) has an upper limit frequency in the region of 0.1 to 0.4 Hz.

4. Apparatus according to one of Claims 1 to 3, characterised in that the evaluation device (9, 10) comprises a bandpass filter (10) for the higher-frequency signal components $S_{HF}$.

5. Apparatus according to Claim 4, characterised in that the bandpass filter (10) for detecting breathing can be adjusted to a frequency range of 0.2 to 0.6 Hz, and in that the upper limit frequency of the low-pass filter (9) is smaller than or equal to 0.2 Hz.

6. Apparatus according to Claim 4 or 5, characterised in that the bandpass filter (10) for measuring the beat volume of the heart is adjustable to a frequency of 1 to 3 Hz.

7. Apparatus according to one of Claims 1 to 6, characterised in that the correction device (11) for correcting the higher-frequency signal components $S_{HF}$ is designed with the low-frequency signal components $S_{NF}$, in accordance with the relation

$$\frac{K1}{S_{NF}}\ -\ K2,$$

K1 being a first variable which takes into account the geometry of the electrode arrangement (5, 6) and K2 being a second variable which takes into account the type of transmission path between the electrodes (5, 6).

8. Apparatus according to Claim 7, characterised in that in the correction device (11) a shaping element (12) which converts the low-frequency signal components $S_{NF}$ according to the relation

$$\frac{K1}{S_{NF}}\ -\ K2$$

is connected on the output side to a device (13 - 15) for forming the maximum/minimum value difference $S_{HF\ max}$ - $S_{HF\ min}$ of the higher-frequency signal components $S_{HF}$ to the effect of forming the correction.

9. Apparatus according to Claim 8, characterised in that the shaping element (12) and the device (13 - 15) for forming the maximum/minimum value difference are connected via a multiplier (16) to the effect of forming the product between the maximum/minimum value difference $S_{HF\ max}$ - $S_{HF\ min}$ and the signal

$$\frac{K1}{S_{NF}}\ -\ K2.$$

6

EP 0 249 825 B1

**10.** Apparatus according to one of Claims 1 to 9, characterised in that from the signal output (17) of the correction device (11) the correction signal $S_k$ is fed as a control signal to the frequency control component (24) of a frequency-controlled heart pace-maker (21) for controlling the stimulation frequency to the effect that when the correction signal $S_K$ changes, the stimulation frequency is correspondingly changed.

**Revendications**

1. Dispositif pour mesurer l'impédance de tissus corporels et comportant une source de signaux (2,19) servant à injecter un signal électrique dans le tissu corporel, un dispositif (1,18) disposé sur le tissu corporel et comportant des électrodes (5,6) et servant à détecter un signal d'impédance $S_I$ à partir du tissu corporel en fonction d'un signal électrique injecté, et un dispositif d'évaluation (9,10), qui, pour l'évaluation du signal d'impédance $S_I$, extrait, de ce signal d'impédance, des composantes à basse fréquence et à fréquence supérieure $S_{NF}$ et $S_{HF}$, et dans lequel, étant donné que le signal d'impédance $S_I$ dépend des variation $\Delta\sigma_R$ de la conductance $\sigma_R$ du tissu corporel et des variations $\Delta K1$ de la géométrie K1 du dispositif à électrodes (5,6), le dispositif d'évaluation (9,10) est agencé de manière à extraire des composantes de signal à basse fréquence ($S_{NF}$), qui dépendent uniquement des variations $\Delta\sigma_R$ de conductance $\sigma_R$, caractérisé par le fait qu'il est prévu un dispositif de correction (11), qui corrige les composantes de signal à fréquence supérieure $S_{HF}$, qui sont proportionnelles à

$$\frac{\Delta K_1}{\sigma_R + \Delta\sigma_R}$$

corrige les composantes au moyen des composantes de signal à basse fréquence $S_{NF}$ dans le sens d'une élimination de l'influence des composantes de signal à fréquence supérieure $S_{HF}$ par les variations $\Delta\sigma_R$ de la conductance et comporte une sortie (17) pour le signal corrigé $S_A$.

2. Dispositif selon la revendication 1, caractérisé par le fait que le dispositif d'évaluation (9,10) comprend un filtre passe-bas (9) pour les composantes de signal à basse fréquence $S_{NF}$, qui dépendent des variations $\Delta\sigma_R$ de la conductance.

3. Dispositif suivant la revendication 2, caractérisé par le fait que le filtre passe-bas (9) possède une fréquence limite supérieure, située dans la plage de 0,1 à 0,7 Hz.

4. Dispositif suivant l'une des revendications 1 à 3, caractérisé par le fait que le dispositif d'évaluation (9,10) comprend un filtre passe-bande (10) pour les composantes de signal à fréquence supérieure $S_{HF}$.

5. Dispositif suivant la revendication 4, caractérisé par le fait que le filtre passe-bande (10) peut être réglé pour détecter l'aspiration dans une plage de fréquences allant de 0,2 jusqu'à 0,6 Hz et que la fréquence limite supérieure du filtre passe-bande (9) est inférieure ou égale à 0,2 Hz.

6. Dispositif suivant la revendication 4 ou 5, caractérisé par le fait que le filtre passe-bande (10) peut être réglé à une fréquence comprise entre 1 et 3 Hz, pour une mesure de l'amplitude du battement cardiaque.

7. Dispositif suivant l'une des revendications 1 à 6, caractérisé par le fait que le dispositif de correction (11) est agencé de manière à corriger les composantes de signal à fréquence supér $S_{HF}$ avec les composantes de signal à basse fréquence $S_{NF}$ conformément à la relation

7

$$\frac{K1}{S_{NF}} - K2,$$

K1 étant une première grandeur tenant compte de la géométrie du dispositif à électrodes (5,6) et K2 une seconde grandeur tenant compte du type de la voie de transmission entre les électrodes (5,6).

8. Dispositif suivant la revendication 7, caractérisé par le fait que dans le dispositif de correction (11), un circuit de conversion (12), qui convertit les composantes de signal à basse fréquence $S_{NF}$ conformément à la relation

$$\frac{K1}{S_{NF}} - K2,$$

est interconnecté au côté sortie d'un dispositif (13-15) servant à former la différence valeur maximale/valeur minimale $S_{HF\,max}$ - $S_{HF\,min}$ des composantes du signal à fréquence supérieure $S_{HF}$, en vue de l'exécution de la correction.

9. Dispositif suivant la revendication 8, caractérisé par le fait que le circuit de conversion (12) et le dispositif (13-15) servant à former la différence valeur maximale/valeur minimale sont raccordés, par l'intermédiaire d'un multiplexeur (16) pour la formation du produit de la différence valeur maximale/valeur minimale $S_{HF\,max}$ - $S_{HF\,min}$ et du signal

$$\frac{K1}{S_{NF}} - K2.$$

10. Dispositif suivant l'une des revendications 1 à 9, caractérisé par le fait que la sortie (17) du signal du dispositif d'évaluation (11) envoie le signal de correction $S_k$ en tant que signal de commande à la partie (4) de commande de la fréquence d'un stimulateur cardiaque (21) dont la fréquence est commandée, pour réaliser la commande de la fréquence de stimulation de manière à modifier de façon correspondante cette fréquence de stimulation dans le cas où le signal de correction $S_K$ varie.

FIG 1

FIG 2

FIG 3